# EUROPEAN PATENT APPLICATION

(11) **EP 1 342 480 A1**
(43) Date of publication of application: **10.09.2003**
(21) Application number: 03005119.7
(22) Date of filing: 07.03.2003
(51) Int. Cl.: A61M 1/16

(54) **Assembly for fastening and using a salt cartridge in a dialysis machine**

(30) Priority: 08.03.2002 IT BO20020118
(71) Applicant: BELLCO S.p.A., 20121 Milano (IT)
(72) Inventor: Berti, Maurizio, 41038 S. Felice Sul Panaro (IT); Facchini, Mauro, 41037 Mirandola (IT)
(74) Representative: Cerbaro, Elena, Dr.

(57) **Abstract**

An assembly (1) for fastening and using a salt cartridge (2) in a dialysis machine, and having two connecting members (3, 4) for connecting the salt cartridge (2) to a dialysis fluid preparation circuit or a disinfectant fluid circuit; and two supporting arms (10, 11) fixed to a supporting structure (12), and each having a respective fastening portion (13) for releasably engaging a respective connecting member (3, 4).

## Description

The present invention relates to an assembly for fastening and using a salt cartridge in a dialysis machine.

Basically, dialysis consists in bringing a patient's blood (which, in uremic subjects, is a solution rich in waste substances for elimination and substances for rebalancing) into contact with a solution of known composition via a semipermeable membrane. The latter solution, hereinafter referred to as the "dialysis solution", contains none of the substances (urea, creatine, uric acid, phosphorous, etc.) which are to be eliminated completely from uremic blood, but does contain other substances (sodium, potassium, calcium, magnesium, etc.) at sufficient concentrations to achieve a given desired balance in the blood.

The dialysis solution is normally prepared in large quantities and stored in special vessels until needed. The main problem posed by storing the dialysis solution is one of space, on account of the volume and number of vessels to be stored at the dialysis facility. Secondly, the solution stock must be safeguarded against external contamination, even for relatively long periods, which means obvious contamination hazards and/or additional storage cost.

To eliminate the above drawbacks, dry salt cartridges have been devised, by which to prepare the dialysis solution when needed, by adding deionized water to the cartridge to obtain a concentrated solution, which is then diluted as required to form the dialysis solution.

Such cartridges are fixed to the dialysis machine by means of a fastening device, which comprises two arms, each comprising a connecting member for fastening a respective end of the cartridge. More specifically, each connecting member has a first end, which, in use, is inserted inside the cartridge; and a second end connected to a tube, so as to feed deionized water into the cartridge and draw from the cartridge the concentrated salt solution later forming the dialysis solution.

Obviously, after each dialysis, all the equipment which has and will again come into contact with the dialysis solution must be disinfected, which means also flushing the connecting members with disinfectant fluid.

To disinfect the cartridge fastening device as easily and effectively as possible, two main systems have been devised. A first is described in Patent Application EP 0278100, and comprises a top and bottom arm, both hinged to a supporting surface, and which assume a position perpendicular to the supporting surface when the cartridge is loaded, and a position parallel to and against the supporting surface when disinfecting the fastening device. When the arms are positioned against the supporting surface, the connecting members engage two respective spouts projecting from the supporting surface, and are connected to a disinfectant fluid circuit.

A second system is described in US Patent 6,036,858, and comprises a bottom arm fixed to a supporting structure; and a top arm, which is parallel to the bottom arm, slides to and from the bottom arm along a guide formed in the supporting structure, and, to disinfect the fastening device, is slid towards the bottom arm to connect the two connecting members hermetically. In both the above systems, each of the two arms has an inner channel forming part of the main circuit, and along which flows either the distilled water and concentrated solution when preparing the dialysis solution, or the disinfectant solution when disinfecting the fastening device.

Though both provide for effectively sterilizing the cartridge fastening device, the above systems are highly complex, particularly in terms of maintenance. In particular, both systems feature a fluid circuit inside the fastening device, with the obvious drawbacks in terms of circuit monitoring and maintenance.

It is an object of the present invention to provide a dialysis machine salt cartridge fastening device designed to provide a straightforward, low-cost solution to the problems posed by the known state of the art.

According to the present invention, there is provided an assembly for fastening and using a salt cartridge in a dialysis machine, characterized by comprising two connecting members for connecting said salt cartridge to a dialysis fluid preparation circuit or a disinfectant fluid circuit; and two supporting arms fixed to a supporting structure, and each having a respective fastening portion for releasably engaging a respective connecting member.

In a preferred embodiment of the present invention, the assembly for fastening and using a salt cartridge comprises a fitting which cooperates with both the connecting members at the disinfecting stage.

In a further preferred embodiment of the present invention, the assembly for fastening and using a salt cartridge comprises a lock member hinged to the fitting and for locking one of the two connecting members when disinfecting the assembly.

The assembly as defined above is both extremely straightforward and effective. In fact, the arms of the assembly according to the invention serve solely to support the cartridge and the connecting members fitted to it, and comprise no portion of the dialysis or disinfectant fluid circuit. As such, the part of the circuit involving the cartridge is not incorporated in any structure, thus enabling effective circuit monitoring and easy maintenance. Moreover, the supporting arms and connecting members being physically separate, replacing a particular component part of the assembly is easier and cheaper than with known devices.

A non-limiting embodiment of the present invention will be described by way of example with reference to the accompanying drawings, in which:
Figure 1 shows a schematic view, with parts removed for clarity, of a preferred embodiment of the assembly at the dialysis solution preparation stage;
Figure 2 shows a schematic view, with parts removed for clarity, of the Figure 1 assembly at the disinfecting stage;
Figure 3 shows a larger-scale detail of the Figure 1 assembly.

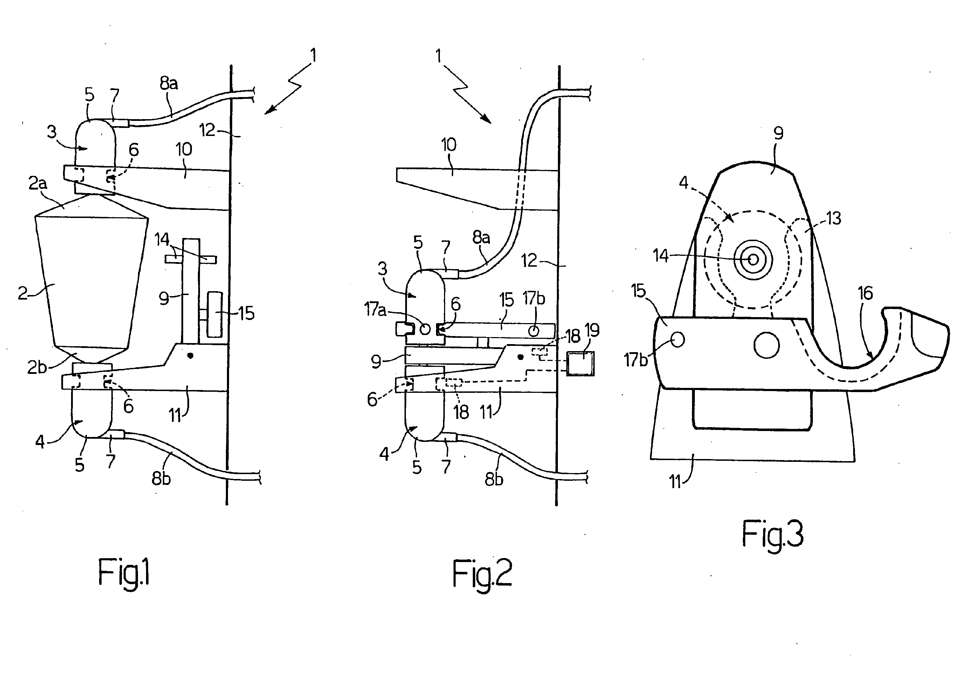

Number 1 in Figures 1 and 2 indicates as a whole an assembly for fastening and using a salt cartridge 2 in a dialysis machine.

Assembly 1 comprises two connecting members 3 and 4, each of which is dome-shaped and defines a convex outer wall 5, which has a circular groove 6, and on which is formed an outer attachment 7 for a respective tube 8a, 8b. Both tubes 8a and 8b connect assembly 1 to a fluid circuit for either preparing a dialysis solution (Figure 1) or recirculating a disinfectant fluid (Figure 2).

Each connecting member 3, 4 has a concave inner wall not visible in the accompanying drawings, and where a known inner attachment (not described) is formed for connection to either cartridge 2 or a fitting 9 described below.

Assembly 1 comprises a top supporting arm 10 and a bottom supporting arm 11, which are fixed firmly to a supporting structure 12, and each of which comprises a fork-shaped portion 13 (Figure 3) for engaging a respective circular groove 6. More specifically, the thickness of fork-shaped portion 13 is less than the height of circular groove 6, so that cartridges of different lengths can be used, by fork-shaped portion 13 engaging different parts of circular groove 6, while at the same time ensuring effective grip.

It should also be pointed out that, when assembling assembly 1, top supporting arm 10 can be fitted to supporting structure 12 at different distances from bottom supporting arm 11, thus making assembly 1 even more versatile in terms of cartridge size.

A fitting 9 is hinged to bottom supporting arm 11, and has two male portions 14 communicating with each other and located on opposite sides of fitting 9. Each male portion 14 engages a respective inner attachment of a connecting member 3, 4 during disinfection; and fitting 9 is hinged to bottom supporting arm 11 so that, during dialysis (Figure 1), in which connecting members 3 and 4 are fitted to cartridge 2, fitting 9 swings clear into a vertical rest position.

A lock member 15 is hinged to fitting 9, has a C-shaped portion 16, and provides for locking connecting member 3, when connecting members 3 and 4 are connected to fitting 9 (Figure 2), by C-shaped portion 16 engaging circular groove 6.

Connecting member 3 and lock member 15 house, respectively, a first and a second magnet 17a and 17b, which, when lock member 15 locks connecting member 3 connected correctly to fitting 9, are located at two proximity sensors 18 housed in bottom arm 11 and connected to a known microprocessor 19 shown schematically in Figure 2. Proximity sensors 18 interact with magnets 17a and 17b to indicate correct positioning of connecting member 3 and lock member 15, while at the same time ensuring correct disinfection.

In actual use, when preparing the dialysis solution (Figure 1), connecting members 3 and 4 are connected to a top end 2a and a bottom end 2b of cartridge 2 respectively. Once fitting 9 is set to the vertical rest position, the whole defined by cartridge 2 and connecting members 3 and 4 is fixed to supporting structure 12 by means of supporting arms 10 and 11, the fork-shaped portions 13 of which engage circular grooves 6 in the two connecting members 3 and 4. The deionized water is fed along tube 8a connected to connecting member 3, through which it flows into cartridge 2, and the concentrated salt solution formed flows out of cartridge 2 through connecting member 4 and along tube 8b to the dialysis solution preparation circuit.

When dialysis is completed, the cartridge is disposed of, but parts such as connecting members 3 and 4 and tubes 8a and 8b must be disinfected. At which point, fitting 9 is positioned horizontally, and one of the two male portions 14 engages the inner attachment of connecting member 4; connecting member 3 is detached from supporting arm 10 and also fitted to fitting 9 and locked by lock member 15 as described above and shown in Figure 2; disinfectant fluid is then fed along tubes 8a and 8b, and necessarily also flows through connecting members 3 and 4; and magnets 17 and proximity sensors 18 ensure correct positioning of connecting member 3.

The assembly for fastening and using a salt cartridge in a dialysis machine according to the present invention obviously solves the problems posed by the known state of the art by physically separating the supporting arms from the connecting members, thus simplifying maintenance and improving the efficiency of the assembly. Moreover, the relative dimensions of circular groove 6 and fork-shaped portion 13 enable cartridges of different lengths to be used, thus making the assembly according to the invention fairly versatile.

Finally, if necessary, the assembly according to the invention enables the cartridge and connecting members to be detached from the supporting arms to shake the solution formed inside the cartridge.

## Claims

1. An assembly (1) for fastening and using a salt cartridge (2) in a dialysis machine, **characterized by** comprising two connecting members (3, 4) for connecting said salt cartridge (2) to a dialysis fluid preparation circuit or a disinfectant fluid circuit; and two supporting arms (10, 11) fixed to a supporting structure (12), and each having a respective fastening portion (13) for releasably engaging a respective connecting member (3, 4).

2. An assembly as claimed in Claim 1, **characterized in that** each of said connecting members (3, 4) comprises a convex outer wall (5) having a circular groove (6); an outer attachment (7) for a respective tube (8) being formed on said outer wall (5).

3. An assembly as claimed in Claim 2, **characterized in that** said fastening portion is a fork-shaped portion (13) engaging said circular groove (6).

4. An assembly as claimed in Claim 3, **characterized in that** said fork-shaped portion (13) is of a thickness smaller than the height of said circular groove (6).

5. An assembly as claimed in any one of the foregoing Claims, **characterized by** comprising a fitting (9) having two fitting portions (14) connected to each other and each connectable to a respective connecting member (3, 4).

6. An assembly as claimed in Claim 5, **characterized in that** said fitting portions are two male portions (14).

7. An assembly as claimed in Claim 5 or 6, **characterized in that** said fitting (9) is hinged to one of said supporting arms (10, 11).

8. An assembly as claimed in Claim 7, **characterized in that** said fitting (9) is hinged to a bottom supporting arm (11).

9. An assembly as claimed in any one of Claims 2 to 8, **characterized by** comprising a lock member (15) having a lock portion (16) for engaging said circular groove (6) of one of said connecting members (3, 4) connected to said fitting (9).

10. An assembly as claimed in Claim 9, **characterized in that** said lock member (15) is hinged to said fitting (9).

11. An assembly as claimed in Claims 8 and 9, **characterized by** comprising a first magnet (17a) and a second magnet (17b) housed in said connecting member (3) and said lock member (15) respectively; said bottom supporting arm (11) comprising two proximity sensors (18) connected to a microprocessor (19) and cooperating with said magnets (17a, 17b).
